# EUROPEAN PATENT SPECIFICATION

(11) **EP 2 078 096 B1**
(45) Date of publication and mention of the grant of the patent: **01.09.2010**
(21) Application number: 07818603.8
(22) Date of filing: 01.10.2007
(51) Int. Cl.: C12Q 1/68

(54) **METHOD FOR THE DETECTION OF AN ACTIVATION OF THE IMMUNE SYSTEM OR THE EXTENT OF CELL DEATH**
VERFAHREN ZUM NACHWEIS EINER AKTIVIERUNG DES IMMUNSYSTEMS ODER DES AUSMASSES VON ZELLTOD
MÉTHODE DESTINÉE À LA DÉTECTION D'UNE ACTIVATION DU SYSTÈME IMMUNITAIRE OU DE L'ÉTENDUE DE LA MORT CELLULAIRE

(30) Priority: 29.09.2006 US 827571 P
(43) Date of publication of application: 15.07.2009
(73) Proprietor: LEUKOCARE AG, 82152 Martinsried / München (DE)
(72) Inventor: MARGRAF, Stefan, 60318 Frankfurt (DE)
(74) Representative: Vossius & Partner
(86) International application number: PCT/EP2007/008523
(87) International publication number: WO 2008/037499

(56) References cited:
- GUPTA ET AL: "Induction of Neutrophil Extracellular DNA Lattices by Placental Microparticles and IL-8 and Their Presence in Preeclampsia" HUMAN IMMUNOLOGY, NEW YORK, NY, US, vol. 66, no. 11, November 2005 (2005-11), pages 1146-1154, XP005353842 ISSN: 0198-8859
- UMETANI NAOYUKI ET AL: "Prediction of breast tumor progression by integrity of free circulating DNA in serum." JOURNAL OF CLINICAL ONCOLOGY : OFFICIAL JOURNAL OF THE AMERICAN SOCIETY OF CLINICAL ONCOLOGY 10 SEP 2006, vol. 24, no. 26, 10 September 2006 (2006-09-10), pages 4270-4276, XP002469075 ISSN: 1527-7755
- AHN S J ET AL: "PicoGreen quantitation of DNA: effective evaluation of samples pre- or post-PCR." NUCLEIC ACIDS RESEARCH 1 JUL 1996, vol. 24, no. 13, 1 July 1996 (1996-07-01), pages 2623-2625, XP002469076 ISSN: 0305-1048
- FATOUROS IOANNIS G ET AL: "Cell-free plasma DNA as a novel marker of aseptic inflammation severity related to exercise overtraining." CLINICAL CHEMISTRY SEP 2006, vol. 52, no. 9, 13 July 2006 (2006-07-13), pages 1820-1824, XP002469077 ISSN: 0009-9147
- LAM NICOLE Y L ET AL: "Time course of early and late changes in plasma DNA in trauma patients." CLINICAL CHEMISTRY AUG 2003, vol. 49, no. 8, August 2003 (2003-08), pages 1286-1291, XP002469078 ISSN: 0009-9147
- ZHONG X Y ET AL: "Elevation of both maternal and fetal extracellular circulating deoxyribonucleic acid concentrations in the plasma of pregnant women with preeclampsia." AMERICAN JOURNAL OF OBSTETRICS AND GYNECOLOGY FEB 2001, vol. 184, no. 3, February 2001 (2001-02), pages 414-419, XP002469079 ISSN: 0002-9378
- BRINKMANN VOLKER ET AL: "Neutrophil extracellular traps kill bacteria." SCIENCE (NEW YORK, N.Y.) 5 MAR 2004, vol. 303, no. 5663, 5 March 2004 (2004-03-05), pages 1532-1535, XP002469080 ISSN: 1095-9203 cited in the application

## Description

The present invention relates to a method for the detection of an activation of the immune system, preferably in the sense of NET formation, or of the extent of cell death in a non-tumorous tissue or in a body fluid, wherein non-cell-bound DNA is measured in a sample from an individual as characterized in the claims.

After major operations, in particular with a heart-lung-machine, but also after accidents with polytraumas, sepsis, burn injuries as well as after ischemia/reperfusion disease (after arterial or capillary occlusion) strong, sometimes excess activations of the immune system are observed. These can cause temporary or permanent damage in organs but can also lead to death. The cellular component of this immune response is mediated by neutrophil granulocytes. To estimate the effects of the activation, it is important to be informed about the extent of the events in a timely manner. Typically, measurements of known indicators or markers are carried out. Nevertheless, until now, only factors are known the activation or expression of which is delayed by one to two or three days.
Thus, the common parameter C-reactive protein (CRP) is increased only about two days after an event, whereas IL-6 is increased after about one day. CRP is induced by IL-6 in the liver and reaches its maximum only 72 hours after induction. Furthermore, in adults, CRP is a very inert parameter the rise of which persists for a long time, does not indicate the short-term therapeutic success and has only very limited application for the detection of inflammatory complications in the early postoperative phase because of its rise caused by the operation. Nevertheless, at present, CRP is the "gold standard" of inflammatory diagnostics and is the absolutely most expensive parameter in most laboratory statistics of clinics with maximum service. Accordingly, markers known so far are not suitable for acute events which require a quick reaction of the attending physician. For example, after arterial occlusion it has to be decided within hours if an amputation is to be carried out.

With 50 to 80% neutrophil granulocytes form the major part of the white blood cells and form the first line of defense against pathogens. They contain granula in their cytoplasm which themselves contain a number of enzymes that can actively kill microorganisms. Neutrophil granulocytes are inter alia activated by the presence of microbes and migrate to the source of infection. Afterwards, the respective contaminants are internalized by phagocytosis and killed with the help of the enzymes from the granula. Recently, a further defense mechanism applied by neutrophils has been discovered: NETs (neutrophil extracellular traps) form a DNA-based structure decorated with different proteins. Protein of azurophil (primary) granulae, e.g. elastase, cathepsin G and myeloperoxidase, can be found as well as proteins specific for secondary and tertiary granulae, such as lactoferrin and gelatinase. Also, histones H1, H2A, H2B, H3 and H4 can be found in the DNA which is the main structural component (Brinkmann, et. al. 2004) showing potent antimicrobial properties. NETs are secreted by neutrophil granulocytes already ten minutes after activation. The release depends on the dose of the activator and thus on the extent of activation. NETs associate both with Gram-positive and Gram-negative bacteria. Together with elastase present in this structure they actively contribute to the inactivation of bacterial virulence factors. Recently, the activity of NETs against an eukaryotic microorganism, i.e. the yeast Candida albicans, beside that against prokaryotic microorganisms has also been shown (Urban et al., 2006). NETs are formed both *in vitro* and *in vivo* where they could be detected in the course of experimental shigellosis in rabbits as well as in spontaneous appendicitis in humans.
The prior art indicates that the fact that NETs present histones extracellularly could also turn out to be harmful for the host body in the context of autoimmune diseases, such as e.g. Lupus erythematosus since these usually not exposed antigens could be recognized as foreign.

The measurement of DNA in the blood for examining the effectiveness of cancer therapeutics is a relatively new but in the meantime regularly applied method. Since cancer therapy aims at the death of the tumor cells, this method for the measurement of the DNA liberated during cell death stands to reason (Umetani et al., 2006).

As already described above, after operative invasions for the detection of inflammations in the body, accidents, it is essential to quickly estimate the extent of an activation of the immune system which has taken place or will take place in order to precociously take the respective counter measures. Therefore, it is desirable to find and to make detectable a marker which provides quickly utilizable results in appropriate methods. It would also be advantageous to have such an indicator for cell death, which in this context means necrosis, after certain pathological events. Despite the growing understanding of the molecular mechanisms underlying the immune response and necrosis, the art has so far not succeeded in finding markers which promptly indicate the extent of damage by an excessive immune response.

The solution of the above-described technical problem is described by the embodiments characterized in the claims.

The present invention relates to a method for the detection of an activation of the immune system, preferably in the sense of NET formation, or the extent of cell death in a non-tumorous tissue or bodv fluid, wherein non-cell-bound DNA is measured in a sample from an individual as characterized in the claims.

An "activation of the immune system" in general is present if certain markers specific therefore can be detected on the cell surface of cells mediating the immune response. An activation is present, inter alia, when significant changes of the cell numbers of the "big" blood count arise, in particular those of the neutrophil granulocytes and lymphocytes. Among other things, immune responses are caused by pathogens in the form of microorganisms, viruses and also by irritant or poisonous substances (example: aspiration pneumonia), allergens, medicaments as well as biologicals (CD28, TeGenero). More specifically, the term "activation of the immune system" denotes the increased release of NETs by neutrophil granulocytes.

Cell death commonly comprises programmed cell death (physiological cell death, apoptosis) or pathological cell death (necrosis).

Apoptosis can be caused from the outside (e.g. by immune cells) or by internal processes in the cell (e.g. after a strong damage of the genetic information). Contrary to necrosis, apoptosis is actively conducted by the cell concerned and forms a part of the metabolism of the cell. Accordingly, this form of cell death is subject to strict control and it is ensured that the respective cell perishes without damaging the neighboring tissue.
Possibly, damaging influences such as e.g. poisons, bacteria, deficits in nutrients or oxygen, radioactivity, can direct cells to necrosis. This is followed by an inflammation reaction of the neighboring tissue. Depending on the kind of tissue, the necrosis completely heals due to the fact that the cells grow again or the necrotic part of the tissue is replaced by a cicatrice formed by connective tissue.
Optically, apoptosis and necrosis are easily distinguishable. Whereas, in the case of apoptosis, a shrinkage of the cell is initiated and DNA is degraded into defined pieces by endonucleases, the cell swells in the case of necrosis leading to the destruction of its plasma membrane. The aftereffect are local inflammations since the cytoplasm, the cell organelles and the DNA are released into the extracellular space and then disposed off by macrophages. In this context, also the DNA from the cell nucleus is released.

"Non-tumorous" in this context means that the tissue has not developed in the course of a change in the genetic information leading to pathological proliferation. The tissue is neither a benign nor a malign cancer. Until now, there was no reason to carry out measurements of non-cell-bound DNA in an individual which is not afflicted with cancer. Until now, the release of non-cell-bound DNA has been known and utilized exclusively in the context of the death of tumor cells in cancer therapies.

Body fluids are all liquids present in the body or which are secreted by the body, e.g. blood, liquor, urine, serous liquids, saliva or pathologically altered stool.

In the course of the present invention, it has surprisingly been found that the concentration of non-cell-bound DNA in the blood rises in the presence of certain pathological events which are not caused by cancer or pathogens. Without wishing to be bound to certain theories, the Applicant acts on the assumption that, depending on the pathological state, this non-cell-bound DNA is present in free form and/or in the form of the above-described NETs. In this case, these NETs are not only produced for the defense of microorganisms but also generally released in the course of immune responses which are caused by certain pathological events. Whereas parts of these NETs are stuck to the capillaries, another part is torn off as a consequence of the blood stream and is present in the plasma and serum. Since, as described above, until now appropriate markers for the precocious detection of such an immune response are missing, the detection of NETs which are already released ten minutes after activation of the NET producing granulocytes in samples of patients can lead to timely reactions of the attending physician which can save lives.

Generally, an individual with a concentration of free DNA in the blood in the range of 0 to about 150 ng/ml is regarded as a healthy individual.

Accordingly, in a preferred embodiment of the method of the present invention, a value significantly higher than 150 ng/ml is indicative of an activation of the immune system or an increased extent of cell death.

With the method of the present invention, activations of the immune system caused by certain events can be timely measured and the individual having undergone such an event can be monitored in order to be able to timely take appropriate measures if an activation of the immune system or an increased extent of cell death is measured. Exemplary events are operation, accident with polytraumas, soft part traumas, ischemia/reperfusion disease, infarction, ischemia, embolism, infection, sepsis, transplantation, poisoning, eclampsia, side effects of medication and/or transfusion.

Preferably, e.g. in case of operations, a measurement of the DNA concentration according to the method of the present invention is carried out prior to the respective event to obtain a comparative value.

If this is not possible, e.g. in the case of accidents, several measurements can be carried out after the event to monitor the condition of the individual.

Preferably and if possible, the method of the invention is applied during or after the above events (e.g. operations) as well as only after events such as e.g. accidents. Measurements of the DNA concentration with the method of the present invention may be carried out several times and for a time period as long as necessary, i.e. until the values measured indicate that no further activation of the immune system or increased extent of cell death is present or to be expected. Depending on the severity of the individual's condition and the nature of the event, the time period between the measurements can vary from about one hour, e.g. immediately after one or more of the above events, to several hours or one day.
On the example of an operation as event, the DNA concentration may be measured immediately prior to the operation to obtain a comparative value. Further measurements are made during the operation to monitor the effects of the operational intervention on the immune system of the individual. Afterwards, measurements can be made e.g. 1 hour, 2 hours, 5 hours and 10 hours after the operation. If the DNA concentration increases significantly as compared to the previous value, the attending physician can timely decide about appropriate measures to take. Typically, the DNA concentration is expected to rise in the course of an operation as well as during or after the other events mentioned above and to remain constant at an elevated level for a certain, variable time and then to decrease again to indicate that the immune system is not further activated.
In case more than one measurements are effected in samples from one individual, the last value obtained is compared to the previous value measured in a sample from said patient, wherein said previous value may also exceed 150 ng/ml. A significantly higher value as compared to the previous value obtained from the patient can be indicative of a further activation of the immune system or an increased extent of cell death. In this regard, "significantly higher" means values at least 10%, preferably at least 20%, more preferably at least 30%, even more preferably at least 50%, most preferably at least 100% higher than the value(s) previously obtained. For example, if a DNA concentration of 800 ng/ml was previously measured in a patient, a DNA concentration of e.g. 1000 ng/ml measured afterwards is indicative of a further activation of the immune system or an increased extent of cell death.

In another preferred embodiment, also an increase in the DNA concentration to a value below 150 ng/ml is regarded as significant, if the increase is by at least 25%, preferably at least 50%, more preferably at least 75% and most preferably at least 100%. For example, if the first value obtained from one individual is 50 ng/ml and the second one measured afterwards is 120 ng/ml, the resulting value is indicative of an activation of the immune system or an increased extent of cell death, even if the value lies below 150 ngimi.

Qualitatively as well as quantitatively, DNA can be routinely detected in multiple ways. Among them are PCR methods as well as the detection with agents specifically interacting with DNA. Non-cell-bound DNA can be determined with a variety of methods. Besides the measurement of the fluorescence after addition of an intercalating dye, nowadays also the measurement of UV absorption of DNA is routinely applied to determine the concentration. The sample volumes necessary have by now reached the low µl range (e.g. measurement with NanoDrop in the range starting from 2 µl). At the moment, the lower detection limit for DNA with the fluorescence method is at about 50ng/ml.

The method disclosed herein aims at the measurement of non-cell-bound DNA. In this regard, the term "non-cell-bound" comprises DNA present outside of cells which is either pure, i.e. without further additional substances such as e.g. proteins, sugar residues not belonging to the DNA etc., but can as well be loaded with a such agents. "Non-cell-bound DNA" preferably comprises DNA in NET structures or DNA which has been released by other cell death, wherein the DNA is present in body liquids or tissue.

In a preferred embodiment, the measurement of non-cell-bound DNA is quantitative.

The concentration or amount of non-cell-bound DNA to be measured is the DNA amount which can be detected in blood or other body fluids or in tissues. The concentration can be determined as absolute value as well as in the form of an amount per weight or per volume, such as e.g. mg/ml, µg/ml, ng/ml or the respective transformations into other units.

The sample comprises plasma or serum obtained from whole blood.

The term "plasma" means the liquid phase of the blood which has been separated from solid components such as cells (erythrocytes, white blood cells, etc.) and which can still coagulate.

The term "serum" describes the liquid part of the blood which is obtained after coagulation of the blood by separation of the cellular constituents which were mixed with thrombocytes and coagulation factors to form the blood cake.

The measurement of the non-cell-bound DNA comprises the determination of fluorescence emission after addition of a fluorescent dye to the plasma or serum.
Certain substances incorporate into double-stranded and also single-stranded DNA (intercalators). To some extent, the substances only then gain or enhance their fluorescent properties. By accumulating in the DNA and upon irradiation with light of certain wavelengths, the substances emit light of a different wave length which can be quantitatively measured and which correlates with the amount of DNA. Fluorescent dyes can also be different from intercalators and can be brought into contact with the DNA to be measured e.g. by chemical modification. Commonly applied dyes are, for example, Cy3, Cy5 and similar substances.

DNA can well be detected with intercalating fluorescent dyes which, as opposed to the detection of decorating proteins with e.g. specific antibodies, has the advantage of a significant time saving. Taken alone or together with the early detectability of the immune reaction, a powerful and clinically valuable method has been developed with the potential displace the existing and considerably more tedious methods in clinical diagnostics.

In an even more preferred embodiment, the fluorescent dye is Picogreen® (Molecular Probes) or Qant-iT™ (Invitrogen). The dye can be used according to the manufacturer's instructions. Preferably, a suitable amount of Picogreen is diluted, preferably immediately prior to use. Suitable amounts are e.g. 1, 2, 3, 4 or 5 µl Picogreen which can be diluted in e.g. 1 ml PBS, serum, plasma or mixtures thereof or, in case other suppliers are used, appropriately adjusted amounts. If the dye is present in dried form it can be appropriately dissolved.

The measurement of non-cell-bound DNA is carried out on the basis of comparison with at least one or more standards.
A standard curve can be produced with DNA, e.g. from calf thymus, by diluting this DNA of a known concentration in a liquid and then measuring the fluorescence and/or absorption, preferably after addition of a suitable dye such as Picogreen. The value obtained thereby is compared with respective values from a healthy or diseased individual. The healthy individual does not have to be of the same species as the one examined. If available, it is preferred that plasma or serum to determine the value of a healthy individual be taken from the respective individual prior to the event potentially causing the release of (NET-) DNA in the body. More particularly, a sample may be taken from an individual prior to e.g. an operation.

In the easiest case, the standard curve consists of one point in combination with a known slope. This point preferably lies in the range of 0.01 - 4µg/ml DNA, further preferred in the range of 0.1 - 2 µg/ml DNA, even more preferred in the range of 0.2 - 0.6 µg/ml DNA. The standard curve can also consist of two or more points in a range of 0.01 - 10 µg/ml.

The standard curve comprises at least one value starting from a dilution lower than 10µg/ml, preferably lower than 7 µg/ml and particularly preferred lower than 5 µg/ml DNA.

The dilution is carried out with plasma or serum of a healthy individual or with a liquid having optical properties comparable to those of serum or plasma.

The skilled person can easily determine which liquids have suitable optical properties and can be used instead of plasma or serum. Those liquids preferably contain albumin or bilirubin or other substances which are suitable to mimic the optical properties of serum or plasma. The concentrations of both substances may be adjusted to resemble to those of the measured sample. The liquid may also or in addition contain 2 to 5 % NaCl.

The dilution with plasma of a healthy individual serves to create equal measurement conditions for the sample and the DNA standard. The measurement of plasma from a healthy individual on its own generates the zero value which, in this case, can constitute a standard value for a normal DNA concentration in the serum/plasma.

The comparison of the concentration measured with known standards from healthy and diseased individuals with different disease patterns can be essential for the diagnosis and for a decision concerning the treatment and therapy.

In a further preferred embodiment, at least one anticoagulant from the group consisting of citrates, heparins, natural and synthetic active ingredients from leech, enzyme inhibitors and chelating agents are added to the whole blood prior to the measurement.

An anticoagulant is a substance which impairs the coagulation of blood after its addition. These substances are routinely applied to obtain blood amenable to diagnostic purposes.

In a further preferred embodiment, the solid components of the blood are separated from serum or plasma by centrifugation or other means based on gravitation or filtration.

Centrifugation is well known in the art as a method for the separation of solid and liquid blood components. It is suitable both for the separation of coagulated and non-coagulated blood into its solid and liquid components. In general, every method suitable for the separation of solid blood components is utilizable to carry out the present invention.
The separation step also ensures that intracellular DNA is separated from the serum or plasma and thus the sample to be measured so that only non-cell-bound DNA is measured.

In a further preferred embodiment, the activation of the immune system is caused by an operative invasion, an accident with polytraumas, soft part traumas, sepsis, burn injury, ischemia/reperfusion disease, infarction, embolism, infection, sepsis, transplantation, poisoning, eclampsia, side effects of medication and/or transfusion.

Among others, an infection can be caused by bacteria, viruses, parasites, yeasts or fungi.

The present invention describes an increase of the non-cell-bound DNA in the blood in the context of conditions arising inter alia after operative invasions, accidents with polytraumas as well as in case of sepsis.
In this respect, major operative invasions are particularly preferred, especially those necessitating the utilization of an heart-lung-machine (HLM), since an activation of the immune system takes place remarkably often if not always if it is used, as shown in Example 2. The utilization of heart-lung-machinees comprises an ischemic phase in the lungs and heart and necessitates a reperfusion after termination of the invasion in the course of each of both an increased concentration of non-cell-bound DNA was also detected.

A sepsis is a systemic infection (not local anymore), often associated with an out-of-control immune reaction. In general, large amounts of transmitters released in an excess reaction lead to an inflammation of the whole body which is associated, inter alia, with swellings, disturbed blood circulation and oxygen deficiency without being useful for fighting the pathogen. Once essential organs are affected the loss of their functionality arising therefrom can rapidly become the essential limiting factor for the survival of the patients.

As described above, granulocytes release their DNA in a net-like structure to catch and inactivate bacteria and fungi. As shown below by means of examples, the concentration of non-cell-bound DNA also rises in the case of polytraumas, soft part traumas, sepsis, burn injuries as well as after ischemia/reperfusion disease. These injuries cause the release of cytokines which elicit the respective immune response or inflammation reaction. However, also predominantly mechanical stimuli (shockwaves, shearing stress) can cause the formation of NETs. Examples therefore are accidents at high velocity, bullet wounds, defenestration and blood roller pumps. Even if the Applicant does not wish to be bound to theories in this context, he expresses the reasonable assumption that the non-cell-bound DNA mainly consists of NETs released by granulocytes.
Furthermore, the Applicant starts from the assumption that NETs also participate in kidney damage and in arterial occlusion disease (AOD, including myocardial infarction), the latter often after a preceding infection. Among others, this possibly results from blockades which are caused by the large surface DNA-based NETs in blood vessels with small diameters. Furthermore, NET formation and adhesion can be caused in vessels with a rheologically unfavorable surface structure e.g. involved in plaque formation, particularly in the presence of high blood pressure which then leads to increased mechanical stress for granulocytes (also formation of biofilms in the case of implants) because of turbulent current in this area.

In a further preferred embodiment the cell death is a consequence of a trauma, burn injury, poisoning, necrosis of liver cells, rhabdomyolysis, operative invasion, accident with polytraumas, soft part traumas, ischemia/reperfusion disease, infarction, ischemia, embolism, infection, sepsis, transplantation, poisoning, eclampsia, side effects of medication and/or transfusion.
In the case of transfusion, an activation of the immune system or cell death may arise if blood of the wrong blood type is transfused.

In the course of these pathological conditions, cell death in the form of necrosis can occur, wherein the DNA of the necrotic cells is released. In general, necrosis is caused by external pathological conditions and, as opposed to apoptosis, occurs uncontrolled.

In a further preferred embodiment, the individual is a mammal.

In a particularly preferred embodiment, the mammal is a human.

In a further preferred embodiment, proteins stick to the DNA.

As described further above, NETs do not consist of pure DNA but are decorated with different kinds of proteins. These proteins are usually proteins found in the nucleus in the environment of the DNA. In the present invention, also DNA-protein-structures which do not correspond to NETs are included.

In a further preferred embodiment, the proteins are proteases or histones.

Basically, the present invention includes all proteins which can serve to specifically detect the presence of NETs or other DNA decorated with proteins, including proteins of azurophil (primary) granulae, e.g. elastase, cathepsin G and myeloperoxidase, as well as proteins specific for secondary and tertiary granula such as lactoferrin and gelatinase.
Accordingly, in a preferred embodiment, the method of the present invention comprises a step of detecting proteins specifically found on NET-DNA and/or other DNA decorated with proteins. Methods for the detection of specific proteins are well known in the art and comprise e.g. Western Blotting, ELISA, FACS analysis or enzymatic assays.

In a further preferred embodiment and in the context of the activation of the immune system the DNA stems from granulocytes.

Also described herein is a method for the preparation of a kit for the detection of an activation of the immune system or of the extent of cell death in an individual, comprising the packaging of a fluorescent dye and a DNA standard in at least one container.

The kit can be utilized if an activation of the immune system in an individual with operative invasions, accidents with polytraumas, soft part traumas, sepsis, burn injuries, ischemia/reperfusion diseases and/or transfusion or cell death after e.g. trauma, burn injury, poisoning, necrosis of liver cells, rhabdomyolysis, infarction, ischemia, embolism, infection, sepsis, operation and/or transfusion occurs. Beside the fluorescent dye and the DNA standard, the kit can furthermore contain plasma of a healthy individual for the dilution of the DNA standard.

Also described herein is a kit for the detection of an activation of the immune system or of the extent of cell death in an individual, comprising a fluorescent dye and a DNA standard. Optionally, the kit can contain plasma of a healthy individual for the dilution of the DNA standard. The various components of the kit may be packaged in one or more containers such as one or more vials. The vials may, in addition to the components, comprise preservatives or buffers for storage.

The figures show:
Figure 1:
   Course of the concentration of free DNA in the blood of patients with polytrauma. The DNA concentration of 26 patients has been tracked daily starting at hospitalization. The level of the DNA concentration correlates with the severity of the injuries and the outcome. Concentrations in µg/ml. Group 1: patients 1 to 12; group 2: patients 13 to 21; group 3: patients 22 to 26. ISS: injury severity score;
Figure 2:
   Comparison of the course of the DNA concentrations of pigs connected to a heart-lung-machine (HLM) with that of control pigs. Sample A: taken prior to the operation; Sample B: taken prior to connection to HLM; Sample B2: taken after 90 min of HLM, prior to reperfusion; Sample C: taken after 30 min reperfusion and the end of the operation. Concentrations in ng/ml.Dark gray: control pigs, light gray: HLM pigs.
Figure 3:
   Course of the DNA concentration in the blood of twelve HLM patients and two off-pump technique patients. Samples taken prior to operation (Sample A2), prior to reperfusion (Sample B), after operation (Sample C), 1 h post operation (Sample D), 1 day after operation (Sample F) and 2 days after operation (Sample G). Concentrations in µg/ml.Dark gray: HLM patients, light gray: patients treated with off-pump technique.

The examples illustrate the invention.

### Example 1

Test procedure for the determination of the DNA concentration in accordance with the present invention.

### Material

**Table 1: List of materials for the conduction of the disclosed methods**

| Description/manufacturer | material/order number. |
|---|---|
| Picogreen ds DNA | Molecular Probes Cat. P-7581 |
| DNA standard from calf thymus | Sigma Cat. D-4810 |
| Dulbecco's PBS w/o Mg/ Ca | PAA Laboratories Mat. H15-002 |
| Clear 96-well Flat Bottom Plate | irrelevant since adjustment of the photometer is modifiable |

### Test procedure

### Preparation:

a) Dilution of the DNA standard from calf thymus (always prepare freshly):
   The standard has a starting concentration of 1 mg/ml. It is applied in the experiment in decreasing dilution starting from 4 µg/ml. For this purpose, it is diluted in sterile-filter EDTA-plasma of a healthy individual (optionally + 30% PBS).
b) Picogreen: Picogreen double-stranded (ds) DNA is stored at -18°C and should be diluted shortly prior to use; for this purpose, 1 to 5 µl Picogreen are diluted in 1 ml PBS.

### Pipetting scheme:

**Table 2: Pipetting scheme for carrying out the disclosed method**

| Patients: | Blank value | Picogreen value |
|---|---|---|
| EDTA-/Citrate-plasma serum | 50 µl | 50 µl |
| PBS w/o Mg/ Ca | 100 µl | - |
| Diluted Picogreen | - | 100 µl |
| Standard: | Blank value for all standard concentrations | Picogreen value |
| diluted standard *(multiple concentrations starting from 4*µ*g*/*ml downwards)* | - | 50 µl |
| EDTA-plasma, which was used for diluting | 50 µl | - |
| PBS w/o Mg/ Ca | 150 µl | - |
| Diluted Picogreen | - | 150 µl |
| Immediate photometric measurement with FUSION photometer (485 nm/reference wave length 530nm) possible! In case of measurement of a series, all plates should be measured with the same measurement parameters (PMT (photomultiplier tubes), light intensity, measurement time, ...) | | |

### Evaluation

The blank value of each patient is subtracted from the Picogreen values. In case of the standard curve only one blank value is prepared for all standard concentrations which is subtracted from all other standard Picogreen values. With the standard curve, a readout of the ds-DNA concentrations of the samples is possible.

### Example 2

Course of the DNA concentration in the blood of patients with different disease patterns after operative invasion associated with polytraumas.

The presence of multiple simultaneously occurred injuries, wherein at least one injury or the combination of multiple injuries is life-threatening, is defined as a polytrauma (definition by Tscherne).

In the medical science, a trauma is a damage, injury or wound caused by force. Multiple injuries (polytrauma) are distinguished from the isolated injury of a single region of the body which can be equally life-threatening, e.g. isolated craniocerebral injury after a shot in the head.

The most frequent causes for polytraumas are traffic accidents and downfalls from high altitude. The care for polytraumatic patients amounts to about 1% of all services of first-aid doctors.

The present study has been carried out with 26 patients whose polytrauma had different causes and who were treated in a hospital. Over the course of their stay in the clinic, blood was regularly collected from the patients and the DNA concentration was determined according to Example 1 from the serum obtained. The patients were divided into three groups according to the course of their condition and the DNA kinetics defined:

| | |
|---|---|
| Group I: | (n=12) Initially (up to 4 hrs after trauma) < 800ng/ml cf-DNA in plasma; Interpretation: low risk, SIRS (systemic inflammatory response syndrome) unlikely |
| Group II: | (n=9) Initially >800ng/ml in plasma with decreasing values <800ng/ml within 72 hrs and remaining low for the next 10 days Interpretation: considerable injury but favourable outcome, initially undistinguishable from III |
| Groups III: | Initially >800ng/ml and/or a subsequent oscillatory course with recurrent values >800ng/ml Interpretation: serious injury, high risk for unfavourable outcome including SIRS and sepsis. |

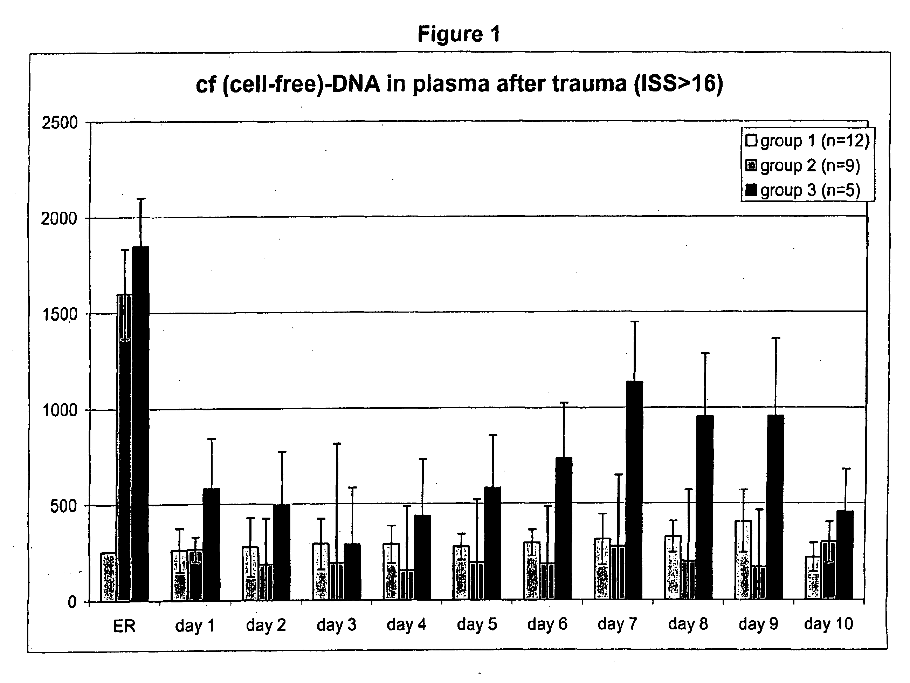

The course of the DNA concentration for each patient is depicted in Figure 1. In many cases, starting from a high DNA concentration directly after the invasion, a differently rapid decrease of the DNA concentration was observed.

During the examination, a correlation of the severity of the injuries and the DNA concentration was observed. Patients 22, 24, 25 and 26 succumbed to their injuries. In all three cases, about 4 to 10 days after having been taken to the hospital, another rise of the DNA concentration of unknown cause of the blood has been observed; however, the value decreased in all cases afterwards. Shortly prior to their death no further increase in the DNA concentration was measured as may possibly have been expected. This can possibly be attributed to the so-called immune paralysis which is an inhibition of the immune reaction the cause of which is not yet exactly understood.

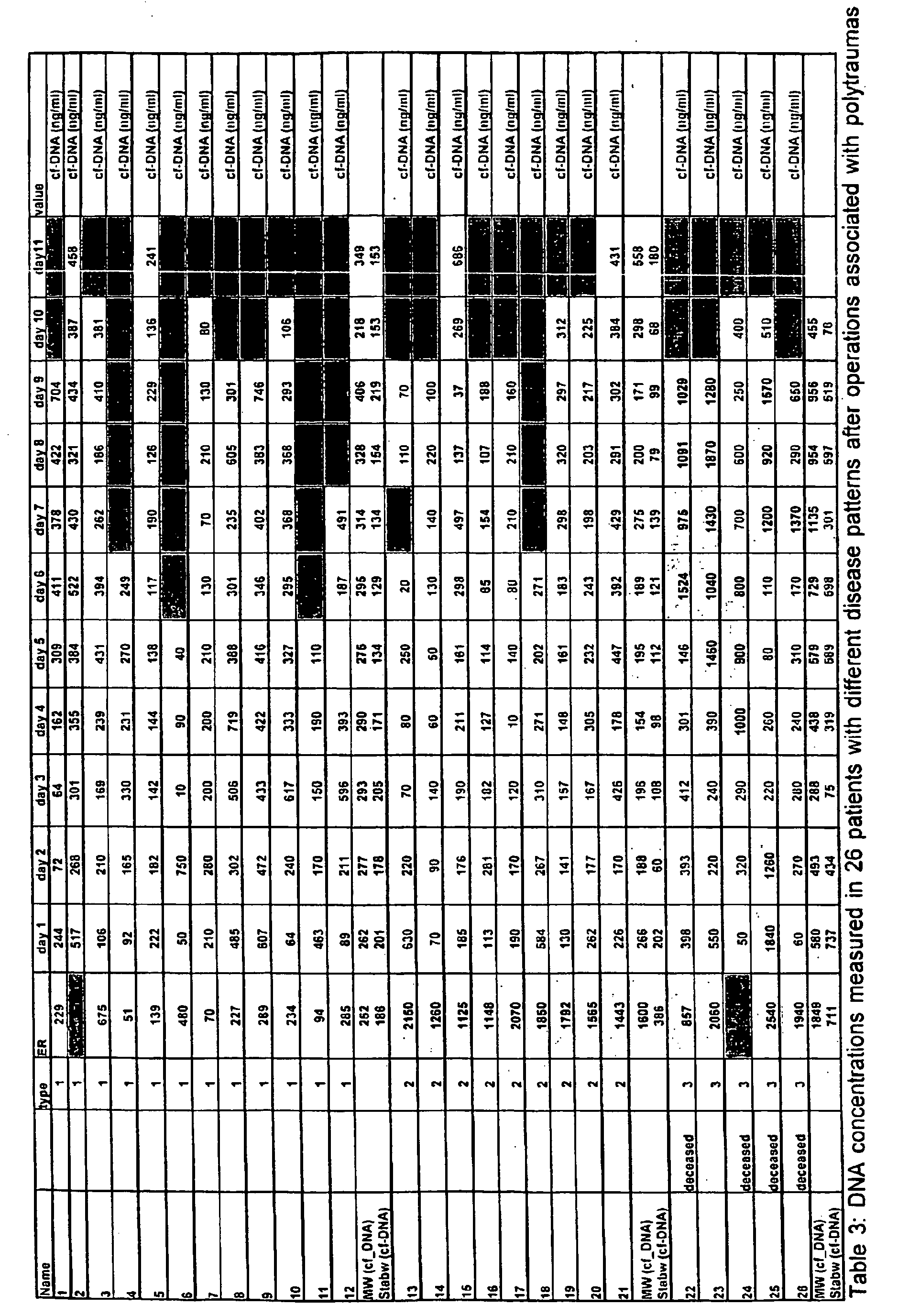

### Example 3

Development of the concentration of free DNA in the blood of pigs after attachment to a heart-lung-machine in comparison to that of control pigs.

Pigs are used as experimental animals for studies as the present one since their anatomy and proportions largely resemble those of humans.

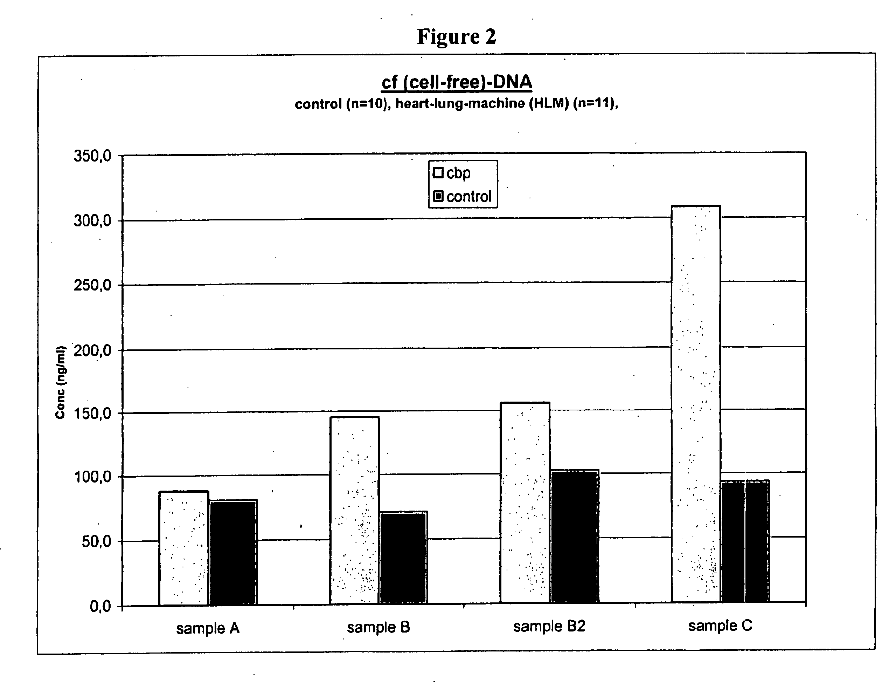

The control group was only thoracotomized and kept in narcosis for two hours. Blood was collected from all pigs prior to operation (A), during the operation (B), 30 min prior to the end of the operation (B2) as well as at the end of the operation (C) from which the concentration of free DNA according to the disclosed method was determined. The measured data of table 4 are depicted in figure 2. Strikingly, the DNA concentration in the blood of the HLM pigs increases three- to sixfold as compared to the starting value in the course of the experiment, whereas that in the blood of the control pig remains constant according to the expectations. The course of the concentration of free DNA in the blood of the septic control pig strikingly resembles that of the HLM pigs.

### Example 4

Comparison of the development of the DNA concentration in the blood of patients who received a bypass and whose blood was conducted through a specific filter during the utilization of a heart-lung-machine and of two patients who received a bypass with the off-pump technique.

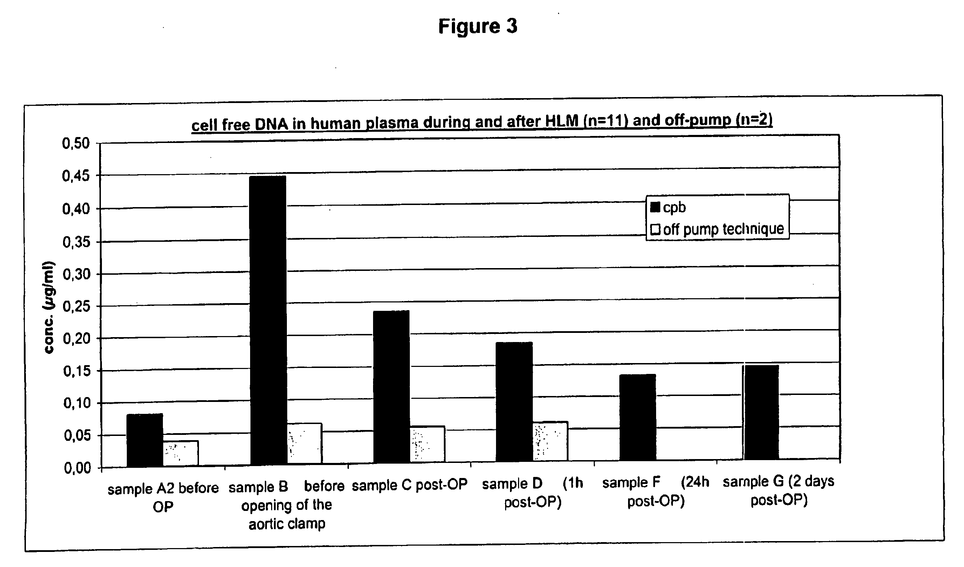

In Figure 3, the results of the study summarized in Table 5 are graphically illustrated. The study revealed that an increased concentration of free DNA was measured in the blood of patients who received a bypass in connection with the use of an HLM. Patients who received a bypass with the off-pump technique, which does not require the connection to an HLM, were previously known to be in a better condition after the operation. The present study confirms that this is correlated with a low concentration of free DNA in their blood.

### References:

Brinkmann, V., Reichard, U., Goosmann, Ch., Fauler, B., Uhlemann, Y., Weiss, D. S., Weinrauch, Y., Zychlinsky, A. (2004). Neutrophil extracellular traps kill bacteria. Science 303; S. 1532-5.
Davis, B. H., Oisen, S. H., Ahead. E., Bigelow, N. C. (2006). Neutrophil CD64 is an improved indicator of infection or sepsis in emergency department patients. Arch Pathol Lab Med 130, S. 654-61.
Umetani, N., Giuliano, A. E., Hiramatsu, S. H., Amersi, F., Nakagawa, T., Martino, S, Hoon, D. S. B. (2006). Prediction of Breast Tumor Progression by Integrity of Free Circulating DNA in Serum. J. Clin Oncol 24, S. 4270-6
Urban, C. F., Reichard, U., Brinkmann, V., Zychlinsky, A. (2005). Neutrophil extracellular traps capture and kill Candida albicans yeast and hyphal forms. Cell Microbiol 8 (4); S. 668-76.

**Table 4: Course of the DNA concentration in pigs connected to a heart-lung-machine (HLM) and a control group. Values in ng/ml; MV: mean value**

| **pig-No.** | **OP-day** | **surgery** **procedure** | **sample A** | **sample B** | **sample B2** | **sample C** | **sample A - Conc.** | **sample B - Conc.** | **sample B2 - Conc.** | **sample C - Conc.** |
|---|---|---|---|---|---|---|---|---|---|---|
| pig06 | 21.07.2006 | **HLM** | 538 | 1360 | | 1764 | **60,9** | **181,7** | | **241,0** |
| pig08 | 03.08.2006 | **HLM** | 416 | 1377 | | 3172 | **43,0** | **184,2** | | **447,9** |
| pig13 | 13.09.2006 | **HLM** | 627 | 676 | 1724 | 3789 | **74,0** | **81,2** | **235,2** | **538,6** |
| pig15 | 21.09.2006 | **HLM** | 486 | 802 | 1706 | 2053 | **53,2** | **99,7** | **232,5** | **283,5** |
| pig16 | 27.09.2006 | **HLM** | 400 | 1943 | 1256 | 2430 | **40,6** | **267,3** | **166,4** | **338,9** |
| pig19 | 05.10.2006 | **HLM** | 1861 | 1438 | 1058 | 2565 | **255,3** | **193,1** | **137,3** | **358,7** |
| pig20 | 11.10.2006 | **HLM** | 1149 | 1339 | 1063 | 3197 | **150,7** | **178,6** | **138,0** | **451,6** |
| pig27 | 01.03.2007 | **HLM** | 217 | 328 | 342 | 420 | **53,5** | **144,2** | **105,4** | **61,1** |
| pig28 | 08.03.2007 | **HLM** | 310 | 286 | 740 | 404 | **57,4** | **52,9** | **137,0** | **74,8** |
| pig39 | 05.07.2007 | **HLM** | 290 | 427 | 786 | 1403 | **52,0** | **77,0** | **141,0** | **253,0** |
| pig40 | 22.08.2007 | **HLM** | 120 | 138 | 111 | 347 | **124,8** | **138,3** | **111,0** | **347,3** |
| | | | | | | MV | **87,8** | **145,3** | **156.0** | **308,8** |
| | | | | | | | | | | |
| pig10 | 17.08.2006 | **control** | 618 | 457 | 573 | 548 | **72,6** | **49,0** | **65,0** | **62,4** |
| pig18 | 04.10.2006 | **control** | 612 | 593 | 377 | 507 | **71,8** | **69,0** | **37,2** | **56,3** |
| pig26 | 14.11.2007 | **control** | 365 | 555 | 313 | 307 | **62,8** | **95,5** | **53,8** | **52,8** |
| pig31 | 23.05.2007 | **control** | 343 | 238 | 218 | 186 | **88,2** | **68,2** | **62,4** | **53,3** |
| pig32 | 23.05.2007 | **control** | 294 | 101 | 172 | 224 | **84,2** | **28,9** | **49,3** | **64,1** |
| pig34 | 31.05.2007 | **control** | 281 | 293 | 294 | 346 | **59,6** | **62,1** | **62,3** | **73,4** |
| pig36 | 14.06.2007 | **control** | 110 | 88 | 246,5 | 285,5 | **48,5** | **38,8** | **108,6** | **125,8** |
| pig41 | 29.08.2007 | **control** | 266 | 311,5 | 323 | 311 | **48,0** | **56,2** | **58,3** | **56,1** |
| pig42 | 30.08.2007 | **control** | 486 | 416 | 440 | 449 | **184,5** | **157,9** | **167,0** | **170,5** |
| pig43 | 06.09.2007 | **control** | 329 | 351 | 1511,5 | 923 | **78,6** | **83,8** | **360,9** | **220,4** |
| | | | | | | MV | 80.9 | 70.9 | 102.6 | 93.5 |

**Table 5: Course of the DNA concentration of HLM patients (patients 1 to 12) and two patients treated with the off-pump technique (patients A and B). Values in µg/ml. The collections were carried out prior to operation (A2), prior to reperfusion (B), directly after operation (C), 1 h post operation (Sample D), 1 day after the operation (Sample F) and 2 days after the operation (Sample G).**

| ***Pat-ID*** | ***OP-date*** | ***sample A2 before OP*** | ***sample B before opening of the aortic clamp*** | ***sample C post-OP*** | ***sample D (1h post-OP)*** | ***sample F (24h post-OP)*** | ***sample G (2 days post-OP)*** |
|---|---|---|---|---|---|---|---|
| Pat. 1 | 24.01.2006 | 0,08 | 0,64 | 0,75 | 0,47 | 0,17 | 0,27 |
| Pat. 2 | 25.01.2006 | 0,15 | 1,26 | 0,3 | 0,18 | 0,09 | 0,26 |
| Pat. 3 | 25.01.2006 | 0,07 | 0,16 | 0,07 | 0,07 | 0,17 | 0,06 |
| Pat. 4 | 30.01.2006 | 0,09 | 0,25 | 0,16 | 0,09 | 0,14 | 0,16 |
| Pat. 5 | 31.01.2006 | 0,08 | 0,31 | 0,25 | 0,17 | 0,14 | 0,05 |
| Pat. 6 | 01.02.2006 | 0,06 | 0,18 | 0,06 | 0,27 | 0,13 | 0,17 |
| Pat. 7 | 22.02.2006 | 0,08 | 0,63 | 0,5 | 0,25 | 0,15 | 0,09 |
| Past. 8 | 20.03.2006 | 0,07 | 0,38 | 0,19 | 0,27 | 0,14 | 0,18 |
| Pat. 9 | 27.03.2006 | 0,07 | 0,19 | 0,04 | 0,07 | 0,06 | 0,18 |
| Pat. 10 | 28.03.2006 | 0,07 | 0,2 | 0,05 | 0,16 | 0,17 | 0,07 |
| Pat. 11 | 10.04.2006 | 0,07 | 0,66 | 0,19 | 0,15 | 0,08 | 0,18 |
| Pat. 12 | 11.04.2006 | 0,075 | 0,47 | 0,26 | 0.06 | 0,15 | 0.07 |
| MW | | ***0,08*** | ***0,44*** | ***0,24*** | ***0,18*** | ***0,13*** | ***0,15*** |
| standard deviation | | **0,02** | **0,32** | **0,21** | **0,12** | **0,04** | **0,08** |
| | | | | | | | |
| Pat A 19.7.07 | | **0,07** | **0,06** | **0,07** | **0,10** | | |
| Pat B 26.7.07 | | **0,01** | **0,06** | **0,04** | **0,02** | | |
| MW | | ***0,04*** | ***0,06*** | ***0,06*** | ***0,06*** | | |

## Claims

1. A method for the detection
a) of an activation of the immune system or
b) the extent of cell death in a non-tumorous tissue or a body fluid,
wherein non-cell-bound DNA is measured in a sample from an individual which comprises plasma or serum obtained from whole blood, wherein the measurement of the DNA comprises the determination of the fluorescence emission after addition of a fluorescent dye to the plasma or serum, wherein the measurement of the free DNA is carried out on the basis of comparison with a standard curve which comprises at least one value starting from a dilution lower than 10 µ/ml DNA and wherein the dilution is carried out with plasma or serum of a healthy individual or with a liquid having optical properties comparable to those of serum or plasma.

2. The method according to claim 1, wherein the measurement of the DNA is quantitive.

3. The method according to claim 1 or 2, wherein the fluorescent dye is Picogreen.

4. The method according to any one of claims 1 to 3, wherein at least one anticoagulant from the group consisting of citrates, heparins, natural and synthetic effective components from leech, enzyme inhibitors and chelating agents is added to the whole blood prior to measurement.

5. The method according to any one of claims 1 to 4, wherein the solid blood components are separated from the serum or plasma by centrifugation or other means based on gravitation or filtration.

6. The method according to any one of claims 1 to 5, wherein the activation of the immune system is caused by an operative invasion, accident with polytraumas, soft part traumas, sepsis, burn injury, infarction, embolism, infection, ischemia/reperfusion disease, transplantation, poisoning, eclampsia, side effects of medication and/or transfusion.

7. The method according to any one of claims 1 to 5, wherein the cell death is caused by burn injury, poisoning, necrosis of liver cells, rhabdomyolysis, operative invasion, accident with polytraumas, soft part traumas, ischemia/reperfusion disease, infarction, ischemia, embolism, infection, sepsis, transplantation, eclampsia, side effects of medication and/or transfusion.

8. The' method according to any one of claims 1 to 7 wherein the individual is a mammal.

9. The method according to claim 8 wherein the mammal is a human.

10. The method according to any one of claims 1 to 9, wherein proteins may be attached to the DNA.

11. The method according to claim 10, wherein the proteins are proteases or histones.

12. The method according to any one of claims 1 to 11, wherein, in addition to the DNA, the proteins according to claim 10 are measured.

13. The method according to any one of claims 1 to 12, wherein the DNA in line with the activation of the immune system stems from granulocytes.

## Patentansprüche

1. Verfahren zum Nachweis
a) einer Aktivierung des Immunsystems oder
b) des Ausmaßes an Zelltod in einem nicht-tumorösen Gewebe oder einer Körperflüssigkeit,
wobei nicht-zellgebundene DNA in einer Probe aus einem Individuum gemessen wird, die Plasma oder Serum aus Vollblut umfasst, wobei die Messung der DNA die Bestimmung der Fluoreszenzemission nach Hinzufügen eines Fluoreszenzfarbstoffes zum Plasma oder Serum umfasst, wobei die Messung der freien DNA auf der Basis eines Vergleichs mit einer Standardkurve durchgeführt wird, die mindestens einen Wert beginnend mit einer Verdünnung von weniger als 10 µg/ml DNA umfasst, und wobei die Verdünnung mit Plasma oder Serum eines gesunden Individuums oder mit einer Flüssigkeit mit optischen Eigenschaften vergleichbar denen von Serum oder Plasma durchgeführt wird.

2. Verfahren nach Anspruch 1, wobei die Messung der DNA quantitativ ist.

3. Verfahren nach Anspruch 1 oder 2, wobei der Fluoreszenzfarbstoff Picogreen ist.

4. Verfahren nach einem der Ansprüche 1 bis 3, wobei mindestens ein Antikoagulans aus der Gruppe bestehend aus Citraten, Heparinen, natürlichen und synthetischen wirksamen Bestandteilen von Blutegel, Enzyminhibitoren und Chelatoren dem Vollblut vor der Messung hinzugefügt wird.

5. Verfahren nach einem der Ansprüche 1 bis 4, wobei die festen Blutbestandteile vom Serum oder Plasma durch Zentrifugation oder andere auf Gravitation oder Filtration basierenden Mittel abgetrennt werden.

6. Verfahren nach einem der Ansprüche 1 bis 5, wobei die Aktivierung des Immunsystems verursacht wird durch einen operativen Eingriff, Unfall mit Polytraumen, Weichteiltraumen, Sepsis, Verbrennungen, Infarkt, Embolie, Infektionen, Ischämie/Reperfusions-Krankheit, Transplantation, Vergiftung, Eklampsie, Nebenwirkungen einer Arzneimittelanwendung und/oder Transfusion.

7. Verfahren nach einem der Ansprüche 1 bis 5, wobei der Zelltod verursacht wird durch Verbrennungen, Vergiftung, Nekrose von Leberzellen, Rhabdomyolyse, operative Eingriffe, Unfall mit Polytraumen, Weichteiltraumen, Ischämie/Reperfusions-Krankheit, Infarkt, Ischämie, Embolie, Infektion, Sepsis, Transplantation, Eklampsie, Nebenwirkungen einer Arzneimittelanwendung und/oder Transfusion.

8. Verfahren nach einem der Ansprüche 1 bis 7, wobei das Individuum ein Säuger ist.

9. Verfahren nach Anspruch 8, wobei der Säuger ein Mensch ist.

10. Verfahren nach einem der Ansprüche 1 bis 9, wobei Proteine an die DNA gebunden sein können.

11. Verfahren nach Anspruch 10, wobei die Proteine Proteasen oder Histone sind.

12. Verfahren nach einem der Ansprüche 1 bis 11, wobei zusätzlich zur DNA die Proteine nach Anspruch 10 gemessen werden.

13. Verfahren nach einem der Ansprüche 1 bis 12, wobei die DNA im Zusammenhang mit der Aktivierung des Immunsystems aus Granulocyten stammt.

## Revendications

1. Méthode de détection
a) d'une activation du système immunitaire ou
b) de l'étendue de la mort cellulaire dans un tissu non-tumoral ou un fluide corporel,
dans laquelle l'ADN non lié à la cellule est mesuré dans un échantillon d'un individu qui comprend du plasma ou du sérum obtenu à partir de l'ensemble du sang, dans laquelle la mesure de l'ADN comprend la détermination de l'émission de fluorescence après l'addition d'un colorant fluorescent dans le plasma ou le sérum, dans laquelle la mesure de l'ADN libre est réalisée sur la base d'une comparaison avec une courbe étalon qui comprend au moins une valeur commençant d'une dilution inférieure à 10µg/ml d'ADN et dans laquelle la dilution est réalisée avec du plasma ou du sérum d'un individu sain ou avec un liquide ayant des propriétés optiques comparables à celles du sérum ou du plasma.

2. Méthode selon la revendication 1, dans laquelle la mesure de l'ADN est quantitative.

3. Méthode selon la revendication 1 ou 2, dans laquelle le colorant fluorescent est du Picogreen.

4. Méthode selon l'une quelconque des revendications 1 à 3, dans laquelle au moins un anticoagulant choisi dans le groupe consistant en citrates, héparines, composants actifs naturels ou synthétiques de la sangsue, inhibiteurs d'enzyme ou agent chélatants est ajouté à l'ensemble du sang avant la mesure.

5. Méthode selon l'une quelconque des revendications 1 à 4, dans laquelle les composants solides du sang sont séparés du sérum ou du plasma par centrifugation ou un autre moyen basé sur la gravité ou la filtration.

6. Méthode selon l'une quelconque des revendications 1 à 5, dans laquelle l'activation du système immunitaire est causée par une invasion chirurgicale, un accident avec polytraumatismes, des traumatismes des tissus mous, une septicémie, une brûlure, un infarctus, une embolie, une infection, une maladie ischémique / de reperfusion, une transplantation, un empoisonnement, une éclampsie, les effets secondaires d'un médicament et/ou d'une transfusion.

7. Méthode selon l'une quelconque des revendications 1 à 5, dans laquelle la mort cellulaire est causée par une brûlure, un empoisonnement, une nécrose des cellules du foie, une rhabdomyolyse, une invasion chirurgicale, un accident avec polytraumatismes, des traumatismes des tissus mous, une maladie ischémique / de reperfusion, un infarctus, une ischémie, une embolie, une infection, une septicémie, une transplantation, une éclampsie, les effets secondaires d'un médicament et/ou d'une transfusion.

8. Méthode selon l'une quelconque des revendications 1 à 7 dans laquelle l'individu est un mammifère.

9. Méthode selon la revendication 8 dans laquelle le mammifère est un humain.

10. Méthode selon l'une quelconque des revendications 1 à 9, dans laquelle des protéines peuvent être fixées à l'ADN.

11. Méthode selon la revendication 10, dans laquelle les protéines sont des protéases ou des histones.

12. Méthode selon l'une quelconque des revendications 1 à 11, dans laquelle, en plus de l'ADN, les protéines selon la revendication 10 sont mesurées.

13. Méthode selon l'une quelconque des revendications 1 à 12, dans laquelle l'ADN en accord avec l'activation du système immunitaire provient de granulocytes.
